# EUROPEAN PATENT APPLICATION

(11) **EP 3 603 675 A1**
(43) Date of publication of application: **05.02.2020**
(21) Application number: 18775569.9
(22) Date of filing: 16.02.2018
(51) Int. Cl.: A61K 47/36, A61K 9/08, A61K 47/02, A61K 47/12, A61K 47/22, A61P 7/08

(54) **PERITONEAL DIALYSIS SOLUTION**

(30) Priority: 31.03.2017 JP 2017072195
(71) Applicant: Terumo Kabushiki Kaisha, Tokyo 151-0072 (JP)
(72) Inventor: USAMI Hirokazu, Nakakoma-gun Yamanashi 409-3853 (JP); NISHITANI Hiroshi, Tokyo 163-1450 (JP); ARIIZUMI Tsuyoshi, Nakakoma-gun Yamanashi 409-3853 (JP)
(74) Representative: Casalonga
(86) International application number: PCT/JP2018/005529
(87) International publication number: WO 2018/179974

(57) **Abstract**

Provided is a sterilized peritoneal dialysis solution which includes an acidic first solution containing icodextrin and a second solution containing 300 to 3,000 mg/L of L-histidine and a pH controlling agent, in which the first solution contains no pH controlling agent, a pH of the first solution after sterilization is 4.0 to 5.3, a pH of the second solution after sterilization is 6.5 to 8.0, and a pH after mixing the first solution and the second solution after sterilization is 6.5 to 7.5.

## Description

### TECHNICAL FIELD

The present invention relates to a peritoneal dialysis solution containing icodextrin.

### BACKGROUND ART

A peritoneal dialysis therapy which is one of the symptomatic therapies for renal failure has attracted attention as one of home medical cares, since a device and apparatus are not large in scale as compared with a dialysis therapy performed through an artificial kidney and also there are few time restrictions. Many of currently-used peritoneal dialysis solutions use glucose as an osmotic substance. Glucose has an advantage of being relatively safe, and being inexpensive. However, a continuous dehydration effect cannot be obtained because glucose has a small molecular weight and is rapidly absorbed from the peritoneum. From the above situation, an investigation of an osmotic substance that can maintain ultrafiltration during long-term storage and can substitute glucose has been carried out, and it has been found that icodextrin, which is a glucose polymer, is suitable for a peritoneal dialysis solution.

Icodextrin is not rapidly absorbed via peritoneum due to a large molecular weight, acts mainly as a colloid osmotic substance, and allows the obtainment of a dehydration effect while maintaining the osmotic pressure with blood plasma. Currently, in a peritoneal dialysis solution using icodextrin, a medical solution is prescribed to be in the range of pH 5.0 to 5.5 in order to prevent the decomposition and coloration of the icodextrin.

According to a recent study, it has been reported that a peritoneal dialysis solution having such a pH substantially impairs the immunological defense mechanism of a peritoneal macrophage, and thus increases the risk of peritonitis due to bacterial penetration. Furthermore, there are also reports of prolonged fever or abdominal pain during an introduction of continuous peritoneal dialysis therapy, and the abdominal pain occurs at the time of injecting the dialysis solution. Furthermore, it has been reported that, with a peritoneal dialysis solution having a pH of 5.0 to 5.5, damage to cultured peritoneal mesothelial cells is significantly high, and it is effective for reducing the damage that the pH of the peritoneal dialysis solution is set at 6.5 or more.

However, the pH of a peritoneal dialysis solution has a significant impact on the stability of icodextrin, and, when the pH is increased without changes, the icodextrin is decomposed into glucose at the time of manufacture or storage and the peritoneal dialysis solution is colored by the deterioration of the glucose, and thus the product value is significantly lowered. That is, the absorbance at 284 nm, which is an index of 5-hydroxymethylfurfural as a main decomposition product of glucose, successively increases. Furthermore, when the pH is increased, the absorbance at 228 nm, which is an index of 3-deoxyglucosone as a main decomposition product of glucose, also increases depending on pH. Furthermore, although there is a method of increasing the pH by using carbonate such as sodium hydrogen carbonate, precipitates are yielded due to a reaction of calcium or magnesium with carbonate under an alkaline condition.

Therefore, as a method of increasing the pH of a peritoneal dialysis solution at the time of administration after suppressing the decomposition and coloration of the icodextrin by lowering the pH, a pharmaceutical preparation has been developed, in which icodextrin and a liquid pharmaceutical component having a high pH are separately stored until a time for use and are aseptically mixed immediately before use (Patent Literature 1).

However, the demand for stability of the peritoneal dialysis solution and safety of the peritoneal dialysis solution has been increasing more and more in recent years, and an emergence of a stable peritoneal dialysis solution having a pH in physiological neutral region that does not adversely affect a human body and suppressing suitably the decomposition to glucose or coloration of icodextrin has been demanded. In addition, there is a demand for a peritoneal dialysis solution having a pH after mixing that is close to a physiological neutral region (specifically, pH 7.0), and also having high stability.

### Citation List

### Patent Literature

Patent Literature 1: JP 2010-150281A (WO 2004-058277, US 2006-128658 A, US 2004-121982 A).

### SUMMARY OF INVENTION

### Technical Problem

The present invention is intended to provide a peritoneal dialysis solution which has enhanced stability of icodextrin during the heat sterilization and the subsequent storage, no precipitation of other components after mixing when the mixing is carried out immediately before administration, a pH close to a physiological neutral region, and high stability of the dialysis solution.

### Solution to Problem

The object described above is achieved according to the following peritoneal dialysis solution of the present invention.

A sterilized peritoneal dialysis solution including an acidic first solution containing icodextrin and a second solution containing 300 to 3,000 mg/L of L-histidine and a pH controlling agent, in which a pH after mixing the first solution and the second solution after sterilization is 6.7 to 7.5.

### DESCRIPTION OF EMBODIMENTS

Hereinbelow, a peritoneal dialysis solution of the present invention will be described in detail. A peritoneal dialysis solution of the present invention contains a first solution mainly containing icodextrin and a second solution containing alkaline components of the peritoneal dialysis solution without containing icodextrin, in which the peritoneal dialysis solution is a two-solution type peritoneal dialysis solution in which the first solution and the second solution are mixed immediately before use, and a pH after mixing is 6.7 to 7.5. Namely, a peritoneal dialysis solution of the present invention is a peritoneal dialysis solution which is very close to neutral (pH 7).

In the peritoneal dialysis solution of the present invention, the content of the icodextrin in the first solution is 60.0 to 94.0 g/L, and preferably 65.0 to 85.0 g/L. In a case in which the content of the icodextrin is less than 60. 0 g/L, proper dialysis cannot be expected in peritoneal dialysis after mixing the first solution and the second solution, and furthermore, in a case in which the content of the icodextrin contained in the first solution exceeds 94.0 g/L, it is not preferable because the amount of the glucose decomposition product resulting from icodextrin becomes large. That is, according to that, the glucose decomposition product resulting from icodextrin during the heat sterilization and the subsequent storage can be suitably suppressed, and an excellent peritoneal dialysis solution allowing proper peritoneal dialysis can be provided.

In the peritoneal dialysis solution of the present invention, a pH of the first solution after sterilization is in an acidic region, and, specifically, it is preferably in the range of pH 4.0 to 5.3, and more preferably in the range of pH 4.5 to 5.3. When the pH is less than 4.0, the amount of 5-hydroxymethylfurfural, which is a glucose decomposition product resulting from icodextrin, becomes large and also the pH after mixing is not in a neutral region, which is preferably 6.7 to 7.5, and thus not desirable. When the pH is more than 5.3, the amount of 3-deoxyglucosone, which is a glucose decomposition product resulting from icodextrin, becomes large and also the pH after mixing is not in a neutral region, which is preferably 6.7 to 7.5, and thus not desirable.

A content of sodium chloride in the first solution is 1.42 to 2.34 g/L, and preferably 1.85 to 2.15 g/L. The sodium chloride is blended for the purpose of adjusting the osmotic pressure, and when the content of sodium chloride in the first solution exceeds 2.34 g/L, it is not preferable because the amount of the glucose decomposition product resulting from icodextrin becomes large.

In the peritoneal dialysis solution of the present invention, from the viewpoint of suppressing a glucose decomposition product resulting from icodextrin, the first solution does not contain an alkaline pH controlling agent.

In the peritoneal dialysis solution of the present invention, the second solution after sterilization is within a neutral to alkaline region, and, specifically, it is preferably within a range of pH 6.7 to 8.0, and more preferably within a range of pH 6.7 to 7.5. When the pH is less than 6.7, or more than 8.0, the pH after mixing will not be 6.7 to 7.5, and therefore not desirable.

The pH can be controlled by a pH controlling agent. As for the pH controlling agent, sodium lactate, trisodium citrate, trisodium phosphate, or the like that are generally used can be used, but sodium hydroxide and sodium hydrogen carbonate are not used.

In the present invention, the second solution contains at least one of sodium chloride, sodium lactate, calcium chloride, and magnesium chloride. A amount of those components is not particularly limited, and it may be contained in the same amount as the amount in an ordinary peritoneal dialysis solution, and it is preferable that sodium chloride is 16.6 to 347.8 g/L, sodium lactate is 21.3 to 448.0 g/L, calcium chloride is 1.22 to 25.7 g/L, and magnesium chloride is 0.24 to 5.10 g/L. From the viewpoint of suppressing the amount of the glucose decomposition product resulting from icodextrin, those components , except sodium chloride, are preferably blended in the second solution containing no icodextrin.

In the present invention, L-histidine (hereinbelow, referred to as "histidine") is contained in the second solution, and a content of the histidine is 300 to 3,000 mg/L, and preferably 600 to 2,000 mg/L. The histidine is blended for the purpose of suppressing the precipitation of calcium and magnesium, and when the content of L-histidine in the second solution is lower than 300 mg/L, pH of the mixture solution decreases over time so that the neutral region cannot be maintained. On the other hand, when the content thereof is higher than 3,000 mg/L, there is a possibility that a symptom caused by subacute toxicity based on histidine is exhibited, and therefore not preferable.

According to the peritoneal dialysis solution of the present invention, the first solution and the second solution are mixed with each other immediately before administration, and the pH is preferably close to pH 7, and, specifically, pH 6.7 to 7.5 is preferable. When the pH after mixing is less than 6.7, the immunological protection mechanism of a macrophage deteriorates or damage to peritoneal mesothelial cells is high, and when the pH after mixing exceeds 7.5, an adverse effect on living bodies is concerned.

According to the peritoneal dialysis solution of the present invention, the first solution and the second solution are separately filled and packaged in a container made of polypropylene, polyvinyl chloride, or the like and sterilized, and the first solution and the second solution are aseptically mixed with each other immediately before being used.

In particular, the peritoneal dialysis solution of the present invention is preferably received in a medical bag having a first chamber and a second chamber formed by being separated by a partitioning means capable of opening the inside thereof, in which the first chamber is provided with a discharge port through which the inside and the outside of the medical bag communicate with each other and separately receives the first solution and the second solution.

An openable partitioning means includes a heat seal which can be broken by a solution pressure of the received first solution or second solution when one of the first chamber and the second chamber is pressed. By doing so, it is possible to easily perform the mixing of the first solution with the second solution. Specific examples of the medical bag include a container of MIDPELIQ (registered trademark) (manufactured by Terumo Corporation) and NICOPELIQ (registered trademark) (manufactured by Terumo Corporation).

In the present invention, as a sterilization method, autoclave sterilization (high pressure steam sterilization) can be mentioned, and the conditions thereof are 110 to 130°C for 25 to 45 minutes, and preferably 115 to 125°C for 30 to 40 minutes.

In general, the peritoneal dialysis solution of the present invention is externally packaged with an oxygen permeable film material such as a three-layer film which is made of polypropylene-polyamide-polypropylene. However, in order to prevent the dialysis solution in the container from deteriorating, it may be further externally packaged with the oxygen impermeable film material.

Examples of the oxygen impermeable film material include a three-layer laminate film containing an ethylene-vinyl alcohol copolymer film, a polyvinyl alcohol film, a polyvinylidene chloride film or the like as an intermediate layer (for example, a laminate film or the like whose outer layer is made of a polyester film, a stretched nylon film, a stretched polypropylene film or the like and inner layer is made of an unstretched polypropylene film), a laminate film containing an aluminum layer (for example, a laminate film made of polyester film-aluminum layer-unstretched polypropylene film, or the like), and a laminate film including an inorganic deposition film (for example, a laminate film composed of polyester film-silicon deposited film-unstretched polypropylene film, stretched nylon film-silicon deposited film-unstretched polypropylene film, polyester film-aluminum deposited film-unstretched polypropylene film, and alumina deposited polyester film-polyvinylidene chloride film-unstretched polypropylene film, or the like), or the like.

Although the peritoneal dialysis solution of the present invention has been described in detail in the above, the present invention is not limited thereto, and various improvements and modifications may be made without departing from the gist of the present invention.

### EXAMPLES

Hereinbelow, the present invention will be described in more detail with reference to specific examples of the present invention. Furthermore, it is to be noted that the present invention is not limited to the following examples.

### (Example 1)

A first solution was prepared by dissolving 75.0 g of icodextrin and 1.87 g of sodium chloride in 840 mL of water for injection. In addition, a second solution was prepared by dissolving 3.48 g of sodium chloride, 4.48 g of sodium lactate, 0.257 g of calcium chloride, 0.051 g of magnesium chloride hexahydrate, and 112 mg of histidine in 160 mL of water for injection. 840 mL of the first solution and 160 mL of the second solution were separately filled in a multi-chamber container made of polypropylene (MIDPELIQ (registered trademark) (manufactured by Terumo Corporation) container), and then added to a three-way bag made of polypropylene/nylon/polypropylene followed by deaeration and packaging. Thereafter, the heat sterilization (121°C, 30 minutes) was carried out by using an autoclave, and then pHs of the first solution, the second solution, and the mixture solution (peritoneal dialysis solution) were measured. pH of the first solution was 4.9, pH of the second solution was 7.5, and pH of the mixture solution was 7.1.

### (Example 2)

A first solution was prepared by dissolving 79.0 g of icodextrin and 1.87 g of sodium chloride in 840 mL of water for injection. In addition, a second solution was prepared by dissolving 3.65 g of sodium chloride, 4.48 g of sodium lactate, 0.257 g of calcium chloride, 0.051 g of magnesium chloride hexahydrate, and 112 mg of histidine in 160 mL of water for injection followed by control of the pH with sodium hydroxide. 840 mL of the first solution and 160 mL of the second solution were filled and sterilized by heating under the same conditions as Example 1, and then pHs of the first solution, the second solution, and the mixture solution (peritoneal dialysis solution) were measured. pH of the first solution was 4.8, pH of the second solution was 7.5, and pH of the mixture solution was 7.1.

### (Example 3)

A first solution was prepared by dissolving 71.0 g of icodextrin and 1.87 g of sodium chloride in 840 mL of water for injection. In addition, a second solution was prepared by dissolving 3.30 g of sodium chloride, 4.48 g of sodium lactate, 0.257 g of calcium chloride, 0.051 g of magnesium chloride hexahydrate, and 224 mg of histidine in 160 mL of water for injection followed by control of the pH with sodium hydroxide. 840 mL of the first solution and 160 mL of the second solution were filled and sterilized by heating under the same conditions as Example 1, and then pHs of the first solution, the second solution, and the mixture solution (peritoneal dialysis solution) were measured. pH of the first solution was 4.8, pH of the second solution was 7.5, and pH of the mixture solution was 7.2.

### (Example 4)

A first solution was prepared by dissolving 75.0 g of icodextrin and 1.87 g of sodium chloride in 840 mL of water for injection. In addition, a second solution was prepared by dissolving 3.65 g of sodium chloride, 4.48 g of sodium lactate, 0.257 g of calcium chloride, 0.051 g of magnesium chloride hexahydrate, and 56 mg of histidine in 160 mL of water for injection followed by control of the pH with sodium hydroxide. 840 mL of the first solution and 160 mL of the second solution were filled and sterilized by heating under the same conditions as Example 1, and then pHs of the first solution, the second solution, and the mixture solution (peritoneal dialysis solution) were measured. pH of the first solution was 5.0, pH of the second solution was 7.3, and pH of the mixture solution was 7.1.

### (Example 5)

A first solution was prepared by dissolving 75.0 g of icodextrin and 1.87 g of sodium chloride in 840 mL of water for injection. In addition, a second solution was prepared by dissolving 3.65 g of sodium chloride, 4.48 g of sodium lactate, 0.257 g of calcium chloride, 0.051 g of magnesium chloride hexahydrate, and 448 mg of histidine in 160 mL of water for injection followed by control of the pH with sodium hydroxide. 840 mL of the first solution and 160 mL of the second solution were filled and sterilized by heating under the same conditions as Example 1, and then pHs of the first solution, the second solution, and the mixture solution (peritoneal dialysis solution) were measured. pH of the first solution was 5.0, pH of the second solution was 7.7, and pH of the mixture solution was 7.4.

### (Comparative Example 1)

A first solution was prepared by dissolving 75.0 g of icodextrin and 1.87 g of sodium chloride in 840 mL of water for injection. In addition, a second solution was prepared by dissolving 3.48 g of sodium chloride, 4.48 g of sodium lactate, 0.257 g of calcium chloride, and 0.051 g of magnesium chloride hexahydrate in 160 mL of water for injection followed by control of the pH with sodium hydroxide. 840 mL of the first solution and 160 mL of the second solution were filled and sterilized by heating under the same conditions as Example 1, and then pHs of the first solution, the second solution, and the mixture solution (peritoneal dialysis solution) were measured. pH of the first solution was 5.0, pH of the second solution was 7.2, and pH of the mixture solution was 6.5.

### (Comparative Example 2)

A first solution was prepared by dissolving 75.0 g of icodextrin and 1.87 g of sodium chloride in 840 mL of water for injection. In addition, a second solution was prepared by dissolving 3.48 g of sodium chloride, 4.48 g of sodium lactate, 0.257 g of calcium chloride, and 0.051 g of magnesium chloride hexahydrate, and 28 mg of histidine in 160 mL of water for injection followed by control of the pH with sodium hydroxide. 840 mL of the first solution and 160 mL of the second solution were filled and sterilized by heating under the same conditions as Example 1, and then pHs of the first solution, the second solution, and the mixture solution (peritoneal dialysis solution) were measured. pH of the first solution was 5.0, pH of the second solution was 7.1, and pH of the mixture solution was 6.6.

### (Comparative Example 3)

A first solution was prepared by dissolving 75.0 g of icodextrin and 1.87 g of sodium chloride in 840 mL of water for injection. In addition, a second solution was prepared by dissolving 3.48 g of sodium chloride, 4.48 g of sodium lactate, 0.257 g of calcium chloride, and 0.051 g of magnesium chloride hexahydrate, and 1 mg of sodium hydroxide in water for injection followed by control of the pH with sodium hydroxide. 840 mL of the first solution and 160 mL of the second solution were filled and sterilized by heating under the same conditions as Example 1, and then pHs of the first solution, the second solution, and the mixture solution (peritoneal dialysis solution) weremeasured. pH of the first solution was 5.0, pH of the second solution was 8.5, and pH of the mixture solution was 7.2.

### (Comparative Example 4)

A first solution was prepared by dissolving 75.0 g of icodextrin and 1.87 g of sodium chloride in 840 mL of water for injection. In addition, a second solution was prepared by dissolving 3.48 g of sodium chloride, 4.48 g of sodium lactate, 0.257 g of calcium chloride, and 0.051 g of magnesium chloride hexahydrate, and 1 g of sodium hydrogen carbonate in 160 mL of water for injection. 840 mL of the first solution and 160 mL of the second solution were filled and sterilized by heating under the same conditions as Example 1, and then pHs of the first solution, the second solution, and the mixture solution (peritoneal dialysis solution) were measured. pH of the first solution was 4.9, pH of the second solution was 7.9, and pH of the mixture solution was 7.2. Furthermore, in the second solution, precipitation of calcium and magnesium occurred from 0 Months.

### (Temporal change test)

For Examples 1 to 5 and Comparative Examples 1 to 4, a temporal change in the pH, calcium, and magnesium in the mixture solution was measured. The results are shown in Tables 1 and 2. Furthermore, storage of the peritoneal dialysis solution was carried out in a constant temperature bath at 40°C.

**[Table 1]**

| pH of mixture solution | | | | |
|---|---|---|---|---|
| Number of days | 0 Months | 1 Month | 2 Months | 3 Months |
| Example 1 | 7.1 | 7.1 | 7.0 | 7.0 |
| Example 2 | 7.1 | 7.1 | 7.0 | 7.0 |
| Example 3 | 7.2 | 7.2 | 7.2 | 7.1 |
| Example 4 | 7.1 | 7.0 | 7.0 | 6.9 |
| Example 5 | 7.4 | 7.3 | 7.3 | 7.2 |
| Comparative Example 1 | 6.5 | 6.4 | 6.4 | 6.4 |
| Comparative Example 2 | 6.6 | 6.6 | 6.5 | 6.5 |
| Comparative Example 3 | 7.2 | 6.4 | 6.4 | 6.4 |
| Comparative Example 4 | 7.2 | 7.3 | 7.3 | 7.4 |

**[Table 2]**

| Calcium concentration (%) in mixture solution | | | | |
|---|---|---|---|---|
| Number of days | 0 Months | 1 Month | 2 Months | 3 Months |
| Example 1 | 100 | 104 | 102 | 103 |
| Example 2 | 100 | 104 | 102 | 103 |
| Example 3 | 100 | 104 | 102 | 103 |
| Example 4 | 100 | 103 | 101 | 101 |
| Comparative Example 1 | 100 | 103 | 101 | 102 |
| Comparative Example 2 | 100 | 104 | 102 | 103 |
| Comparative Example 3 | 98 | 102 | 100 | 101 |
| Comparative Example 4 | 75 | 65 | 55 | 40 |

**[Table 3]**

| Magnesium concentration (%) in mixture solution | | | | |
|---|---|---|---|---|
| Number of days | 0 Months | 1 Month | 2 Months | 3 Months |
| Example 1 | 97 | 101 | 100 | 101 |
| Example 2 | 97 | 101 | 100 | 101 |
| Example 3 | 98 | 101 | 100 | 101 |
| Example 4 | 98 | 101 | 100 | 101 |
| Comparative Example 1 | 97 | 101 | 100 | 100 |
| Comparative Example 2 | 97 | 101 | 100 | 100 |
| Comparative Example 3 | 98 | 101 | 101 | 101 |
| Comparative Example 4 | 69 | 60 | 53 | 49 |

As shown in Table 1, in Comparative Examples 1 and 2, pH of the mixture solution was lower than 6.8 from 0 Months as shown in Table 1. Furthermore, in Comparative Example 3, the calcium and magnesium concentrations were decreased as shown in Tables 2 and 3, because slight precipitation of calcium and magnesium has occurred after mixing (0 Months). After that (1 Month to 3 Months), the pH was decreased as shown in Table 1. In Comparative Example 4, precipitation of calcium and magnesium has occurred from 0 Months, i.e., immediately after the production, and thus the calcium and magnesium concentrations were decreased as shown in Tables 2 and 3.

Namely, once mixing is carried out, the peritoneal dialysis solution of the present invention has a pH close to and no precipitation of calcium and magnesium, and it is also stable for a long period of time after mixing. Specifically, in a constant temperature bath at 40°C, the peritoneal dialysis solution is capable of maintaining the pH at 6.7 to 7.5 for 3 months after mixing.

### Industrial Applicability

As it has been described in detail in the above, the peritoneal dialysis solution of the present invention is a peritoneal dialysis solution which can make the pH of mixture solution to be close to the very much physiological neutral region, specifically pH 7.0, while maximally suppressing the glucose decomposition product resulting from icodextrin during the heat sterilization and the subsequent storage, and also has excellent stability in that state, and thus it can be industrially applied.

The peritoneal dialysis solution of the present invention is as follows.
(1) The present invention is a sterilized peritoneal dialysis solution including an acidic first solution containing icodextrin and a second solution containing 300 to 3,000 mg/L of L-histidine and a pH controlling agent in which a pH after mixing the first solution and the second solution after sterilization is 6.7 to 7.5.
   The peritoneal dialysis solution of the present invention contains a first solution containing mainly icodextrin and a second solution containing neutral to alkaline peritoneal dialysis solution components without containing icodextrin, and it is a two-solution type peritoneal dialysis solution in which the first solution and the second solution are mixed with each other immediately before use, in which the stability of icodextrin during the heat sterilization and the subsequent storage is enhanced, precipitation of other components after mixing does not occur when the mixing is carried out immediately before administration, the pH is close to a physiological neutral region, and the dialysis solution has high stability.
   The peritoneal dialysis solution of the present invention may be also as follows.
(2) The present invention is the peritoneal dialysis solution described in the above (1), in which the sterilized peritoneal dialysis solution includes the acidic first solution containing 60.0 to 94.0 g/L of the icodextrin and 1.42 to 2.34 g/L of sodium chloride and the second solution, the first solution does not contain a pH controlling agent, a pH of the first solution after sterilization is 4.0 to 5.3, a pH of the second solution after sterilization is 6.7 to 8.0, and a pH after mixing the first solution and the second solution after sterilization is 6.7 to 7.5.
(3) The present invention is the peritoneal dialysis solution described in the above (1) or (2), in which the second solution contains at least one of 16.6 to 347.8 g/L of sodium chloride, 21.3 to 448.0 g/L of sodium lactate, 1.22 to 25.7 g/L of calcium chloride, and 0.24 to 5.10 g/L of magnesium chloride.
(4) The present invention is the peritoneal dialysis solution described in the any one of above (1) to (3), in which conditions for the sterilization are 110 to 130°C for 25 to 45 minutes.
(5) The present invention is the peritoneal dialysis solution described in the any one of above (1) to (4), in which the peritoneal dialysis solution is capable of maintaining, in a constant temperature bath at 40°C, a pH at 6.5 to 7.5 for 3 months after mixing.

## Claims

1. A sterilized peritoneal dialysis solution comprising:
an acidic first solution containing icodextrin; and a second solution containing 300 to 3,000 mg/L of L-histidine and a pH controlling agent,
wherein a pH after mixing the first solution and the second solution after sterilization is 6.7 to 7.5.

2. The peritoneal dialysis solution according to claim 1, comprising:
the acidic first solution containing 60.0 to 94.0 g/L of the icodextrin and 1.42 to 2.34 g/L of sodium chloride; and the second solution,
wherein the first solution does not contain a pH controlling agent, a pH of the first solution after sterilization is 4.0 to 5.3, a pH of the second solution after sterilization is 6.7 to 8.0, and a pH after mixing the first solution and the second solution after sterilization is 6.7 to 7.5.

3. The peritoneal dialysis solution according to claim 1 or 2,
wherein the second solution contains at least one of 16.6 to 347.8 g/L of sodium chloride, 21.3 to 448.0 g/L of sodium lactate, 1.22 to 25.7 g/L of calcium chloride, and 0.24 to 5.10 g/L of magnesium chloride.

4. The peritoneal dialysis solution according to any one of claims 1 to 3,
wherein conditions for the sterilization are 110 to 130°C for 25 to 45 minutes.

5. The peritoneal dialysis solution according to any one of claims 1 to 4,
wherein the peritoneal dialysis solution is capable of maintaining, in a constant temperature bath at 40°C, a pH at 6.5 to 7.5 for 3 months after mixing.
